# EUROPEAN PATENT SPECIFICATION

(11) **EP 4 238 562 B1**
(45) Date of publication and mention of the grant of the patent: **17.12.2025**
(21) Application number: 21885419.8
(22) Date of filing: 29.10.2021
(51) Int. Cl.: A61K 31/365, A61K 36/19, A61P 29/00, A61P 31/14

(54) **ANDROGRAPHOLIDE DERIVATIVE FOR USE IN THE TREATMENT OF INFLAMMATORY DISEASES ASSOCIATED WITH A CYTOKINE STORM**
ANDROGRAPHOLIDDERIVAT ZUR VERWENDUNG BEI DER BEHANDLUNG VON ENTZÜNDLICHEN ERKRANKUNGEN IM ZUSAMMENHANG MIT EINEM ZYTOKINSTURM
DÉRIVÉ D'ANDROGRAPHOLIDE DESTINÉ À ÊTRE UTILISÉ DANS LE TRAITEMENT DE MALADIES INFLAMMATOIRES ASSOCIÉES À UN ORAGE CYTOKINIQUE

(30) Priority: 29.10.2020 ES 202031082
(43) Date of publication of application: 06.09.2023
(73) Proprietor: Consejo Superior de Investigaciones Científicas (CSIC), 28006 Madrid (ES); Universitat Politècnica de València, 46022 Valencia (ES); Universidad de Navarra, 31009 Pamplona (Navarra) (ES); Fundación para la Investigación Médica Aplicada, 31008 Pamplona (ES)
(72) Inventor: RIVERO BUCETA, Eva María, 46022 Valencia (ES); BOTELLA ASUNCIÓN, Pablo, 46022 Valencia (ES); VIDAURRE AGUT, Carla, 46022 Valencia (ES); BENLLOCH BAVIERA, Jose María, 46022 Valencia (ES); NOVOA GARCÍA, Beatriz, 46022 Valencia (ES); FIGUERAS HUERTA, Antonio, 46022 Valencia (ES); GONZÁLEZ ASEGUINOLAZA, Gloria, 46022 Valencia (ES); SMERDOU PICAZO, Cristian, 46022 Valencia (ES); PINEDA LUCENA, Antonio A., 46022 Valencia (ES); PRÓSPER CARDOSO, Felipe Luis, 46022 Valencia (ES); ARGEMÍ BALLBÉ, Josepmaría, 46022 Valencia (ES)
(74) Representative: Isern Patentes y Marcas S.L.
(86) International application number: PCT/ES2021/070786
(87) International publication number: WO 2022/090605

(56) References cited:
- CN-A- 101 371 832
- MAURYA VIMAL K ET AL: "Structure-based drug designing for potential antiviral activity of selected natural products from Ayurveda against SARS-CoV-2 spike glycoprotein and its cellular receptor", VIRUSDISEASE, SPRINGER INDIA, INDIA, vol. 31, no. 2, 24 May 2020 (2020-05-24), pages 179 - 193, XP037183120, ISSN: 2347-3584, [retrieved on 20200524], DOI: 10.1007/S13337-020-00598-8
- KHALIFA SHADEN A M ET AL: "Screening for natural and derived bio-active compounds in preclinical and clinical studies: One of the frontlines of fighting the coronaviruses pandemic", PHYTOMEDICINE, ELSEVIER, AMSTERDAM, NL, vol. 85, 29 August 2020 (2020-08-29), XP086537940, ISSN: 0944-7113, [retrieved on 20200829], DOI: 10.1016/J.PHYMED.2020.153311
- CAI WENTAO; CHEN SUNRUI; LI YONGTAO; ZHANG ANDING; ZHOU HONGBO; CHEN HUANCHUN; JIN MEILIN: "14-Deoxy-11,12-didehydroandrographolide attenuates excessive inflammatory responses and protects mice lethally challenged with highly pathogenic A(H5N1) influenza viruses", ANTIVIRAL RESEARCH, ELSEVIER BV, NL, vol. 133, 28 July 2016 (2016-07-28), NL , pages 95 - 105, XP029727969, ISSN: 0166-3542, DOI: 10.1016/j.antiviral.2016.07.020
- PARICHATIKANOND, W. ; SUTHISISANG, C. ; DHEPAKSON, P. ; HERUNSALEE, A.: "Study of anti-inflammatory activities of the pure compounds from Andrographis paniculata (burm.f.) Nees and their effects on gene expression", INTERNATIONAL IMMUNOPHARMACOLOGY, ELSEVIER, AMSTERDAM, NL, vol. 10, no. 11, 1 November 2010 (2010-11-01), NL , pages 1361 - 1373, XP027430059, ISSN: 1567-5769, DOI: 10.1016/j.intimp.2010.08.002
- LIU WEN; FAN TING; LI MANRU; ZHANG GUOHUI; GUO WENJIE; YANG XIAOLING; JIANG CHUNHONG; LI XIANG; XU XIANGYU; TANG ANSHU; LIU KEQIN;: "Andrographolide potentiates PD-1 blockade immunotherapy by inhibiting COX2-mediated PGE2 release", INTERNATIONAL IMMUNOPHARMACOLOGY, ELSEVIER, AMSTERDAM, NL, vol. 81, 1 February 2020 (2020-02-01), NL , XP086095251, ISSN: 1567-5769, DOI: 10.1016/j.intimp.2020.106206
- REHAN MOHD, AHMED FIROZ, HOWLADAR SAAD M., REFAI MOHAMMED Y., BAEISSA HANADI M., ZUGHAIBI TORKI A., KEDWA KHALID MOHAMMED, JAMAL M: "A Computational Approach Identified Andrographolide as a Potential Drug for Suppressing COVID-19-Induced Cytokine Storm", FRONTIERS IN IMMUNOLOGY, vol. 11, 24 June 2021 (2021-06-24), XP055938757, DOI: 10.3389/fimmu.2021.648250

## Description

The present invention relates to the andrographolide AG5 compound for use in the treatment of the inflammatory reaction caused by a cytokine storm, particularly produced by CoViD-19, bacteria with superantigens or by CAR-T, TIL or BiTE cell therapies.

### BACKGROUND OF THE INVENTION

The rapid expansion of the CoViD-19 global pandemic has posed a serious threat to the health systems of countries everywhere due to its high hospitalization and mortality rate. A significant percentage of CoViD-19 patients die from the cytokine storm, generated by an exaggerated inflammatory response of the human body against an infection to which they have no prior immunity. Moreover, many of the patients who survive the disease after their longer or shorter stay in the Intensive Care Unit (ICU) are left with severe pulmonary sequelae due to the cytokine storm.

The pharmaceutical industry has developed several monoclonal antibodies directed at inhibiting inflammatory cytokines. In that sense, Tocilizumab (Roche), Sarilumab (Sanofi), Siltuximab (Eusa Pharma) are interleukin-6 (IL-6) inhibitors; Anakinra (Amgen) is an interleukin-1 (IL-1) inhibitor and Canakinumab (Novartis) is an IL-1β inhibitor. IL-1beta IL-1β controls inflammasome and, therefore, its inhibition is important in the management of inflammation.

Moreover, the pharmaceutical industry has also developed drugs capable of inhibiting intracellular molecular pathways which also have an effect on inflammation control. In that sense, for example, Baricitinib (Olumiant) is a JAK1/JAK2 pathway inhibitor.

Many of the above compounds have been used in clinical trials during the CoViD-19 pandemic to try to reduce the inflammatory response against the SARS-CoV-2 virus.

However, said drugs suffer from various limitations in practice: monoclonal antibodies often present high toxicity that is reflected in the onset of significant side effects; they inhibit only one component of inflammation without addressing the cytokine storm as a whole; they are exclusively aimed at inhibiting the inflammatory response rather than modulating it.

For centuries, Ayurveda (traditional Indian medicine) has been using the active ingredient andrographolide, present in the Indian plant *Andrographis paniculata,* for the treatment of acute respiratory diseases.

The Chinese pharmaceutical industry has developed in the past an injectable drug called Xiyanping. Although the paper by D. Zhang et al., The clinical benefits of Chinese patent medicines against CoViD-19 based on current evidence, Pharm. Res. 157 (2020) 104882, mentions that said drug consists mainly of andrographolide sulfonate, although in reality it is a semi-synthetic preparation containing a mixture of two andrographolide derivatives, 9-dehydro-17-hydro-andrographolide, and 9-dehydro-17-hydroandrographolide-19-yl sulfate, which are structural derivatives of andrographolide different from those presented in this invention.

Xiyanping has also been used as an effective alternative to antibiotics in clinical practice (Q. Li, et al., Xiyanping plus azithromycin chemotherapy in pediatric patients with mycoplasma pneumoniae pneumonia: a systematic review and meta-analysis of efficacy and safety, Evid.-Based Compl. Alt. 2019 (2019) 2346583).

Xiyanping is also effective as an antipyretic and anti-inflammatory (Q.W. Yang, et al., Crystal structure and anti-inflammatory and anaphylactic effects of andrographlide sulphonate E in Xiyanping, a traditional Chinese medicine injection, J. Pharm. Pharmacol. 71 (2) (2019) 251-259).

Likewise, Xiyanping improves respiratory symptoms, inhibits opportunistic bacterial infections, and regulates immune function, with a lower clinical risk, particularly by means of certain hepatic protection, suggesting that it can alleviate the damage produced in the liver by certain drugs during the treatment of CoViD-19 in acute cases (N. Cai, et al., Theoretical basis and effect characteristics of andrographolide against CoViD-19, Chin. Tradit. Herb. Drugs 51 (5) (2020) 1159-1166).

Furthermore, the activity of Xiyanping on the improvement in cases of sepsis in mice by means of suppressing the MAPK, STAT3 and NF-κB signaling pathways, which play an important role in pulmonary diseases, has been reported (W. Guo, et al., Water-soluble andrographolide sulfonate exerts anti-sepsis action in mice through down-regulating p38 MAPK, STAT3 and NF-κB pathways, Int. Immunopharmacol. 14 (4) (2012) 613-619).

Moreover, the possibility of using andrographolide and other phytochemical compounds naturally present in *Andrographis paniculata* as antivirals has been studied by means of computational methods. In that sense, very recently the interaction of andrographolide has been explored by means of computational modeling of molecular docking with specific components of SARS-CoV-2 for the treatment of acute respiratory syndrome associated with CoViD-19. In this context, S. Alagu Lakshmi et al., Ethnomedicines of Indian origin for combating CoViD-19 infection by hampering the viral replication: using structure-based drug discovery approach. J. Biomol. Struct. Dyn. (2020), doi: 10.1080/07391102.2020.1778537) indicate the affinity of bis-andrographolide for the main protease 3CLpro of SARS-CoV-2, which would potentially allow it to inhibit this enzyme.

The andrographolide-main protease 3CLpro of SARS-CoV-2 affinity is also described by S.K. Enmozhi et al., Andrographolide as a potential inhibitor of SARS-CoV-2 main protease: an in silico approach, J. Biomol. Struct. Dyn. (2020), doi: 10.1080107391102.2020.1760136)*.*

On the other hand, D. Sivaraman and P.S. Pradeep, Scope of phytotherapeutics in targeting ACE2 mediated host-viral interface of SARS-CoV2 that causes CoViD-19, doi: 10.26434/chemrxiv.12089730.v1) describe cellular ACE2 receptor blocking by andrographolide.

Additionally, by using computational *docking* and molecular dynamics methods, the use of andrographolide and three structural derivatives (14-deoxy-11,12-didehydro andrographolide, neoandrographolide and 14-deoxy andrographolide) has been evaluated against four SARS-CoV-2 virus targets, including three non-structural proteins (main protease 3CLpro, PLpro and RNA polymerase targeting RdRp RNA) and a structural protein (spike protein (S)), which are responsible for virus replication, transcription and cellular internalization, selecting neoandrographolide as the best inhibitor (N.A. Murugan et al., Computational investigation on Andrographis paniculata phytochemicals to evaluate their potency against SARS-CoV-2 in comparison to known antiviral compounds in drug trials, J. Biomol. Struct. Dyn. (2020), doi: 10.1080/07391102.2020.1777901).

Lastly, in a study by docking on 27 metabolites of plant origin, analyzing the docking thereof with various targets of the virus (main protease, Nsp9 protein, spike receptor domain, spike receptor ectodomain and HR2 domain), andrographolide was not selected as significant against SARS-CoV-2 compared to other compounds (K.F. Azim et al., Screening and druggability analysis of some plant metabolites against SARS-CoV-2: An integrative computational approach, Informatics in Medicine Unlocked 20 (2020) 100367).

Furthermore, andrographolide has been tested as an antiviral in a microarray device containing some of the main proteins of the SARS-CoV-2 virus, obtaining positive results (P. Chen et al., Establishment and validation of a drug-target microarray for SARS-CoV-2, doi: 10.1080/07391102.2020.1777901).

Moreover, the use of andrographolide in combination with melatonin has been suggested as a potentially effective treatment against CoViD-19. (A. Banerjee et al., Crosstalk between endoplasmic reticulum stress and anti-viral activities: A novel therapeutic target for CoViD-19, Life Sciences 255 (2020) 117842)

Therefore, it is critical to develop new drugs that control the inflammatory response in all its aspects, that present a low toxicity and that also have a high bioavailability in the respiratory tract.

### DESCRIPTION OF THE INVENTION

In a first aspect, the present invention relates to the AG5 compound: 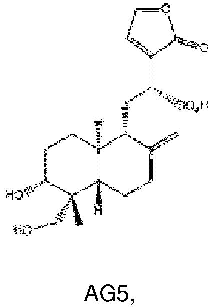 for use in the treatment of a pathology in which an inflammatory reaction associated with a cytokine storm occurs.

The present invention also relates to the salts and solvates of the AG5 compound.

The AG5 compound has chiral centers, which can give rise to various stereoisomers. The present invention relates to each of the individual stereoisomers, as well as to their mixtures.

Throughout the description, the term "cytokine storm" refers to the massive release of inflammation-mediating substances, especially cytokines, as a consequence of a disruption in the immune system produced by an infection or by immunotherapy. This cytokine storm may be caused, among others, by a viral infection, such as the CoViD-19 disease, a bacterial infection, particularly by bacteria with superantigens such as *Staphylococcus aureus* and *Streptococcus pyogenes,* or by immunotherapy, such as CAR-T cell immunotherapy used in cancer treatments, immunotherapy by tumor lymphocytes (TIL) or by bispecific antibodies (BiTE). In that sense, examples of clinical manifestations resulting from a cytokine storm include, among others, systemic inflammatory response syndrome and multiple organ dysfunction syndrome, involving, among other symptoms, shock, fever, rashes, peeling of palms and soles, hypotension, diarrhoea, vomiting, severe myalgia, renal failure, liver problems, respiratory failure, uncontrolled bleeding and disorientation.

Another aspect of the invention relates to the AG5 compound for the use defined above, wherein the cytokine storm is caused by viral infection, bacterial infection or immunotherapy.

In another embodiment, the invention relates to the AG5 compound for the use defined above, wherein the viral infection is CoViD-19.

In another embodiment, the invention relates to the AG5 compound for the use defined above, wherein the bacterial infection is by bacteria with superantigens, and preferably wherein the bacterial infection is by *Staphylococcus aureus* and *Streptococcus pyogenes.*

In another embodiment, the invention relates to the AG5 compound for the use defined above, wherein immunotherapy is carried out by CAR-T cells, by tumor-infiltrating lymphocytes (TIL) or by bispecific antibodies (BiTE), and preferably wherein immunotherapy is carried out by CAR-T cells.

In another embodiment, the invention relates to the AG5 compound for the use defined above, characterized in that it can be administered parenterally or orally.

In another embodiment, the invention relates to the AG5 compound for the use defined above, characterized in that it is administered at a concentration between 0.0001 mg/(kg h) and 10 mg/(kg h) for a time between 1 h and 2000 h, and preferably at a concentration between 0.01 mg/(kg h) and 0.25 mg/(kg h) for a time between 24 h and 480 h.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### BRIEF DESCRIPTION OF THE FIGURES

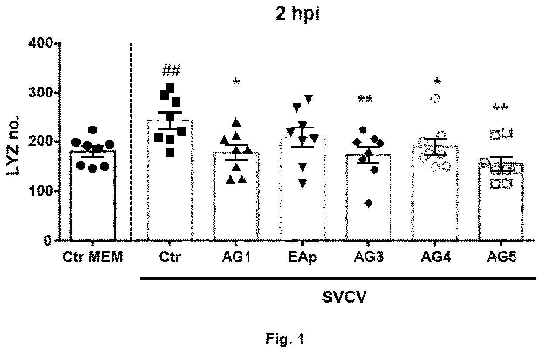

**Figure 1** shows the anti-inflammatory activity of andrographolide (**AG1**), the original extract of *Andrographis paniculata* (EAp), and its structural derivatives (treatment by microinjection) measured based on the count of cells involved in the inflammatory response in *Danio rerio* larvae after infection by microinjection of SVCV (concomitant with drug administration), with respect to control groups that received no treatment. Ctr MEM: control that received an injection of MEM. Ctr: control that received an injection of SVCV. *=statistical differences with respect to the infected control group. hpi: hours post-injection.

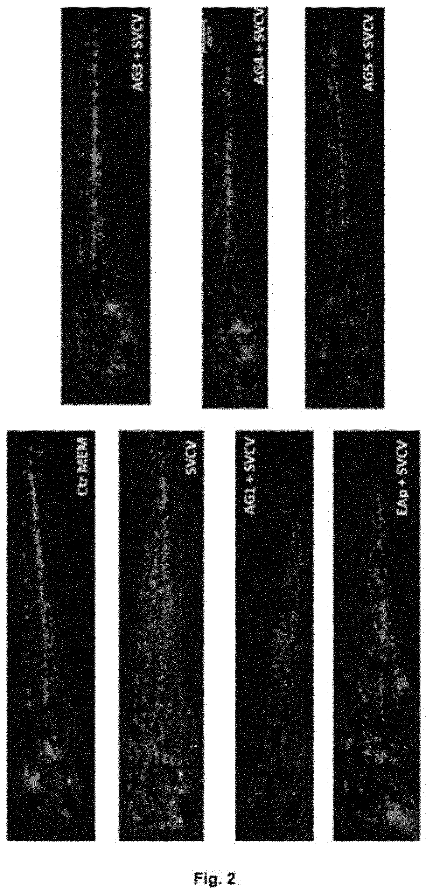

**Figure 2** shows fluorescence micrographs of transgenic *Danio rerio* larvae (LYZ:Red) presenting fluorescent neutrophils after infection by microinjection of SVCV and concomitant treatment with andrographolide (**AG1**), the original extract of *Andrographis paniculata* (EAp) and its structural derivatives. Ctr MEM: control that received an injection of MEM. SVCV: control that received an injection of SVCV.

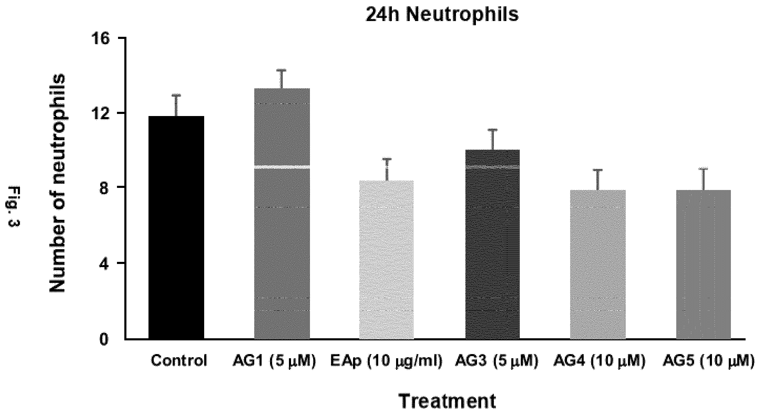

**Figure 3** shows the anti-inflammatory activity of andrographolide (**AG1**), the original extract of *Andrographis paniculata* (EAp) and its structural derivatives (bath treatment) measured based on the migrant neutrophil count under an acute inflammatory stimulus (tail docking) in *Danio rerio* larvae, with respect to a control group that received no treatment. *=statistical differences with respect to the control group.

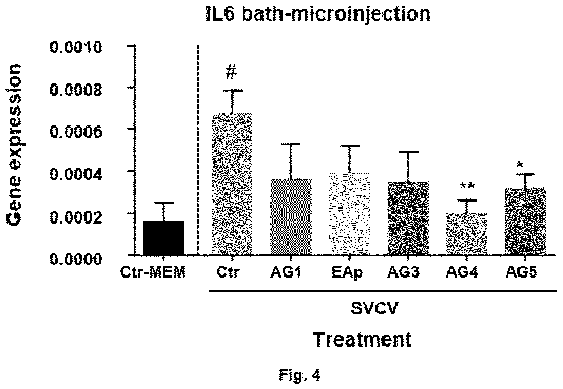

**Figure 4** shows the anti-inflammatory activity of andrographolide (**AG1**), the original extract of *Andrographis paniculata* (EAp) and its structural derivatives (bath treatment) based on IL-6 cytokine gene expression in *Danio rerio* larvae after infection by microinjection of SVCV, with respect to the control group. Ctr MEM: control that received an injection of MEM. Ctr: control that received an injection of SVCV. *=statistical differences with respect to the infected control group.

### EXAMPLES

Next, the invention will be illustrated by means of assays carried out by the inventors which demonstrates the effectiveness and absence of toxicity of the product of the invention.

### EXAMPLE 1: Obtaining andrographolide (3-[2-[decahydro-6-hydroxy-5-(hydroxymethyl)-5,8a-dimethyl-2-methylene-1-naphthalenyl]ethylidene]dihydro-4-hydroxy-2(3H)-furanone) from the extract of Andrographis paniculata

5 g of the crude extract of *A. paniculata* are suspended in 50 mL of Milli-Q^{®} water in a separatory funnel. 500 ml of hexane are added, the mixture is stirred vigorously and left to settle for 1 hour. The organic phase is separated and the process is repeated twice, discarding the n-hexane fractions. Next, 500 ml of chloroform are added. The mixture is stirred vigorously and left to settle for 1 hour. The organic phase is carefully separated and the process is repeated twice, pooling all the chloroform fractions. The solvent is removed under reduced pressure and the oil obtained is diluted with 200 ml of methanol. This solution is heated to a boil, filtered and placed in an ice bath for 1 h. Next, the cold solution is stored in a refrigerator at 4 °C until almost complete evaporation of the solvent. The colorless crystals of andrographolide are washed with cold methanol and dried at room temperature. A 3% yield of andrographolide (compound **AG1**) with a purity greater than 99 % is obtained.

¹H NMR (400 MHz, DMSO) 6.62 (t, *J* = 6.4 Hz, 1H); 5.67 (d, *J* = 5.7 Hz, 1H); 5.01 (s, 1H); 4.91 (s, 1H), 4.81 (s, 1H); 4.62 (s, 1H); 4.39 (dd, *J* = 8.7, 6.4 Hz, 1H); 4.11 (d, *J =* 6.5 Hz, 1H); 4.03 (d, *J =* 9.9 Hz, 1H); 3.85 (d, *J =* 10.6 Hz, 1H); 3.28 - 3.14 (m, 2H); 2.45 (s, 1H); 2.32 (d, *J =* 12.6 Hz, 1H); 2.03 - 1.82 (m, 2H); 1.70 (dd, *J =* 29.9, 14.3 Hz, 4H); 1.35 (dd, *J =* 23.1, 12.7 Hz, 1H); 1.20 (d, *J =* 11 8 Hz, 2H), 1.09 (s, 3H); 0.66 (s, 3H).

¹³C NMR (75 MHz, DMSO) δ 169.89; 147.57; 146.22; 128.95; 108.18; 78.43; 74.27; 64.50; 62.61; 55.47; 54.37; 42.26; 38.56; 37.48; 36.50; 27.87; 23.93; 23.03; 14.71. HR-MS (ESI, m/z) [M+H]⁺ 351.4628 calculated for C₂₀H₂₉O₅, 351.4633 found.

### EXAMPLE 2: Synthesis of 14-deoxy-12(R)-sulfo-andrographolide

1.0 g of andrographolide (**AG1,** 2.9 mmol) is dissolved in 15 ml of 95% ethanol, heating said solution to 50 °C (solution 1). 4.8 ml of H₂SO₄ at 2% (M/M) and 8 ml of water are added to 4 ml of 1M Na₂SO₃ solution (solution 2). Solution 1 is added to solution 2 and the reaction mixture is kept stirring under reflux for 30 minutes. When the reaction is completed, the pH of the reaction is adjusted to pH 6 ~7 by adding sulfuric acid (H₂SO₄) solution at 2% and the solvent is evaporated to dryness. The residue is dissolved in water (20 ml) and extracted with chloroform (20 ml x 3). The solvent is evaporated from the organic phase under reduced pressure. The residue from the aqueous phase is dissolved in methanol (10 ml) and filtered. In the fraction containing the product, the solvent is evaporated under reduced pressure and 0.6 g of the **AG5** compound are obtained (51%).
¹H-NMR (600 MHz, CD₃OD) 7.65 ppm (t, *J =* 1.8 Hz, 1H); 4.95 (o, 2H); 4.87 (o, 2H); 4.66 (S_{b}, 1H); 4.16 (dd, *J* = 10.2 and 6.1 Hz, 1H); 4.05 (d, *J =* 11.4 Hz, 1H); 3.92 (dd, *J =* 12.2 and 1.8 Hz, 1H); 3.30 (t, *J =* 9.8 Hz, 1H); 3.27 (d, *J* = 11.4 Hz, 1H); 2.36 (m, H); 2.31 (dd, *J* =12.6 and 11.4 Hz, 1H); 2.08 (t, *J* = 12.6 Hz, 1H); 1.86 (m, H); 1.83 (m, H); 1.80 (m, H); 1.71 (m, 2H); 1.38 (d_{b}, *J =* 11.8 Hz, 1H); 1.28 (qd, *J* =12.6 and 4.2 Hz, 1H); 1.12 (s, 3H); 1.10 (dd, *J* = 12.6 and 2.4 Hz, 1H); 1.02 (m, H); 0.68 (s, 3H);
¹³C-NMR (150 MHz, CD₃OD) 177.2; 152.1; 149.1; 132.5; 109.2; 81.7; 73.3; 65.8; 57.2; 56.8; 55.3; 44.5; 40.8; 40.1; 38.9; 29.8; 28.0; 26.1; 24.2; 16.4.
HR-MS (ESI, m/z) [M-H]⁻ 413.1639 calculated for C₂₀H₂₉O₇S; 413.1634 found.

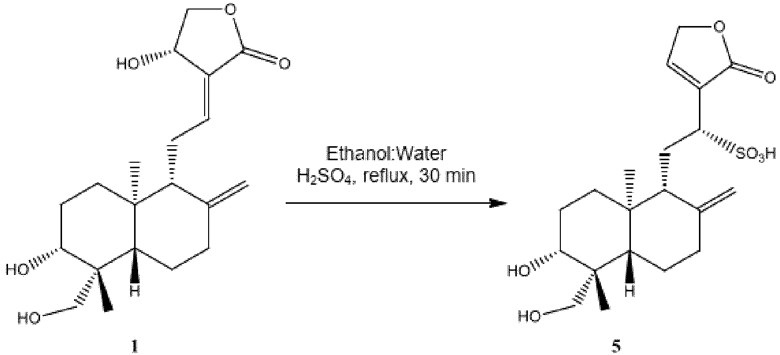

### EXAMPLE 3: Validation of the anti-inflammatory activity of andrographolide and its derivatives in zebrafish (Danio rerio) larvae

Anti-inflammatory activity studies of the compounds were carried out in wild type fish, determining the expression levels of the pro-inflammatory cytokines IL-1β and IL-6. Transgenic larvae with labeled fluorescent neutrophils (Tg(Mpx:GFP)i114) or fluorescent myeloid cells (Tg(LYZ:Red)) were also used to assess how different treatments affect the inflammatory response of these cells. The inflammatory stimulus used was both the virus itself, which causes activation of the inflammasome and death of macrophages by pyroptosis (Varela et al. J. Virol. 2016, 88, 12026), as well as an acute stimulus of inflammation generated by a wound in the tail. The migration of fluorescent leukocytes from these transgenic lines was studied by means of microscopy and image analysis.

In a first assay, the cellular response to inflammation was quantified in transgenic *Danio rerio* larvae (LYZ:Red) after an infection with SVCV treated or untreated (control) with the **AG1** compounds (28 µM), **AG3** (10 µM), **AG4** (10 µM), **AG5** (10 µM) and EAp (10 µg/ml). The treatments were carried out by microinjection (2 nl in the duct of Cuvier) of the drugs in fish 3 days post-fertilization (5-10 fish/well), concomitantly administering the virus and the different compounds. The experiment was carried out analyzing the cells of each fish individually using 8 replicates per treatment (*n* = 8). At 2 h post-injection (2 hpi), the number of cells involved in the inflammatory response was determined. Figure 1 shows that the **AG1, AG3, AG4** and **AG5** compounds significantly reduce the cellular inflammatory response induced by the viral infection with respect to the control groups. Figure 2 shows representative images of fish with each of the treatments.

In a second assay, the effect of the different compounds on an acute inflammatory response produced by a wound was analyzed. To that end, transgenic fish with fluorescent neutrophils (Tg(Mpx:GFP)i114) were used and their migration to the affected area was analyzed. The **AG1** (5 µM), **AG3** (5 µM), **AG4** (10 µM), **AG5** (10 µM) compounds and EAp (10 µg/ml) were administered by bath and after 24 hours the wound was made by cutting the end area of the tail. After 24 hours, neutrophils that migrated to the area of the wound were counted. It was observed that the AG4 and AG5 compounds significantly decreased the inflammatory response generated by the migration of neutrophils to the wound, contributing to the resolution of inflammation. Figure 3.

In a third and final assay, the gene expression of proinflammatory cytokines was determined in *Danio rerio* (wild type) larvae treated with the **AG1** (5 µM), **AG3** (5 µM), **AG4** (10 µM), **AG5** (10 µM) compounds and EAp (10 µg/ml), and subsequently infected with SVCV. The treatments were carried out in a bath for 24 h on fish 1 day post-fertilization (10 fish/well). The experiment was carried out in quadruplicate (*n* = 4). On the second day post-fertilization each fish was injected (2 nl in the duct of Cuvier) 5x10⁴ TCID50/mL of SVCV. At 24 h, the expression of IL-6 was determined by qPCR. The results obtained in Figure 4 indicate that the **AG4** and **AG5** compounds significantly reduce IL-6 production in infection by SVCV with respect to untreated control groups.

### EXAMPLE 4: Validation of the absence of toxicity of AG-5 in a repeated dose study in Wistar rats

As part of the preclinical toxicity evaluation, the purpose of this study was to define the maximum repeated dose (MRD) of AG-5, formulated as a solution in PBS (3 mg/ml), in a mammalian rodent species under good laboratory practice (GLP) and to provide data of toxicological interest. To this end, a two-phase study was designed: i) in phase I, the product was administered intravenously in a single dose and following OECD guidelines 425 (OECD Guidelines for the testing of chemicals. Guidelines 425: Acute oral toxicity: up and down procedure. Adopted 3 October 2008*),* to a total of 12 8-week old female Wistar rats by means of a dose escalation process (incremental factor 3.2), the maximum tolerated dose (MTD) was provisionally determined; ii) in phase II, the animals (a total of 30 8-week old Wistar rats, 15 males and 15 females) were given a daily intravenous administration for 7 days and the MRD was confirmed.

The initial dose to be used in phase I was selected from the sequence of doses included in OECD guidelines 425, that is, 5.5, 17.5 and 30 mg/kg of body weight. The doses to be tested in phase II were determined based on the results obtained in phase I, selecting 3 doses from the dose interval lower than that in which serious toxic effects would have been observed, that is, 15, 20 and 30 mg/kg/day, with 30 mg/kg being the maximum feasible dose (MFD), in application of the ICH M3(R2) guideline (ICH Harmonized Tripartite Guideline. Non-Clinical Safety Studies for the Conduct of Human Clinical Trials and Marketing Authorization for Pharmaceuticals M3 (R2). Current Step 5 version dated 11 June 2009*).* The study considered the complete assessment of the symptoms, that is, intensity, time of onset and reversibility, the study of analytical (hematological and biochemical) parameters and the determination of the weight of toxicity target organs, to be fundamental.

The conclusions of this study are the following:
- In relation to the intravenous administration of AG-5 in PBS (3 mg/ml) in a single dose:
   1.- It did not produce lethality in any animal, so the LD₅₀ estimated in rats is greater than 30 mg/kg of body weight. This dose should be considered the MTD level in rats in a single dose.
   2.- None of the doses tested in phase I compromised the life of the animal, nor did it produce signs of toxicity related to the administration of AG-5. Based on these results, the dose of 30 mg/kg was considered the reference dose for carrying out phase II.
- In relation to the intravenous administration of AG-5 in PBS (3 mg/ml) at repeated doses, that is, 7 days:
- 3.- The daily administration, for 7 days, at doses of 15, 20 and 30 mg/kg, intravenously in rats did not produce mortality or alterations in the general symptoms. No toxicologically relevant changes in analytical parameters or macroscopic alterations in abdominal and thoracic organs or in the absolute or relative weight of the organs were observed.
   4.- In view of these results, the 30 mg/kg dose is identified as the MRD in this animal species.

### EXAMPLE 5: Validation of the absence of toxicity of AG-5 in a repeated dose study in New Zealand rabbits

As part of the preclinical evaluation of toxicity, the purpose of this study was to define the MRD of AG-5, formulated as a solution in PBS (3 mg/ml), in a non-rodent mammalian species, that is, rabbit, under GLP and to provide data of toxicological interest.

To this end, a two-phase study was designed: i) in phase I, the product was administered intravenously in a single dose and following OECD guidelines 425, to a total of 4 New Zealand rabbits (2 males and 2 females) of about 2.5 kg and 12-13 weeks old, and the maximum tolerated dose (MTD) range was provisionally determined; ii) in phase II, the animals (a total of 4 New Zealand rabbits of about 2.5 kg and 12-13 weeks old) were given a daily intravenous administration for 7 days and the MRD that will serve as a reference (highest dose to be used) was confirmed.

The initial dose to be used in phase I was selected based on the data obtained by the laboratory after the administration of AG-5 in rats at a repeated dose of up to 30 mg/kg, for 7 days. In this study, the dose of 30 mg/kg was established as the MRD to be used in subsequent general toxicity studies. To that end, the dose of 15 mg/kg was selected; coinciding with: i) the extrapolation to rabbits of the 30 mg/kg tested in rats, in application of the body surface area factor; and, ii) the MFD, based on the solubility of the product in PBS (3 mg/ml), and at the maximum volume of administration in rabbits (0.5 ml/100 g body weight). The dose to be tested in phase II was determined based on the results obtained in phase I, selecting a dose from the dose interval lower than that at which serious toxic effects would have been observed, with a maximum of 15 mg/kg/day as MFD, in application of the ICH M3(R2) guideline. The study considered the complete assessment of the symptoms, that is, intensity, time of onset and reversibility, the study of analytical (hematological and biochemical) parameters and the determination of the weight of toxicity target organs, to be fundamental.

The conclusions of this study are the following:
- In relation to the intravenous administration of AG-5 in PBS (3 mg/ml) in a single dose:
   1.- It did not produce lethality in any animal, so the LD₅₀ estimated in rabbits is greater than 15 mg/kg of body weight. This dose should be considered the MTD level in rabbits in a single dose.
   2.- No associated signs of toxicity were observed. Accordingly, and taking into account its condition of maximum possible dose according to solubility, a dose of 15 mg/kg/day was considered as the reference dose for performing phase II.
- In relation to the intravenous administration of AG-5 in PBS (3 mg/ml) at repeated doses, that is, 7 days:
   3.- The daily administration, for 7 days at a dose of 15 mg/kg, intravenously in rats did not produce mortality or alterations in the general symptoms. No toxicologically relevant changes in analytical parameters or macroscopic alterations in abdominal and thoracic organs or in the absolute or relative weight of the organs were observed.
   4.- In view of these results, the 15 mg/kg dose is identified as the MRD in this animal species.

### EXAMPLE 6: Validation of the absence of AG-5 genotoxicity by means of the Ames test

The aim of the study was to characterize the possible genotoxic effect of the assay product AG-5 by means of the Ames test (Ames et al. Proc. Nat. Acad. Sci. USA 1973, 70, 783). To that end, it was determined in a study carried out under GLP whether AG-5 has the ability to produce gene mutations, either by substitution or by mismatch, in *Salmonella typhimurium* bacteria histidine (His-) auxotrophs.

The study was carried out with strains TA98, TA100, TA102, TA1535 and TA1537 in the presence and in the absence of metabolic activation (S9 mixture). The doses recommended by the OECD guidelines were tested: 100 mg/ml, that is, equivalent to 5000 µg/plate, and 5 other lower concentrations (33.3; 11.1; 3.7 and 1.2 mg/ml). No precipitation of the product or toxicity was observed. In view of these results, the mutagenicity test was performed starting from the maximum recommended dose for soluble and non-toxic products (100 mg/ml, corresponding to 5000 µg/plate) and 4 lower doses in 1/3 dilutions. The results were negative in the 5 bacterial strains (TA98, TA100, TA102, TA1535 and TA1537) both in the presence and in the absence of metabolic activation.

The conclusion of the study is that the AG-5 test product did not induce gene mutations under the conditions of this assay, neither in the presence nor in the absence of metabolic activation.

## Claims

1. The AG5 compound: 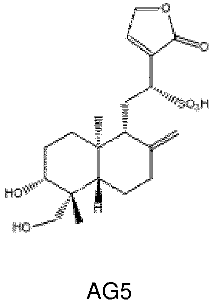 for use in the treatment of a pathology in which an inflammatory reaction associated with a cytokine storm occurs.

2. The AG5 compound for use according to claim 1, wherein the cytokine storm is caused by viral infection, bacterial infection or immunotherapy.

3. The AG5 compound for use according to claim 2, wherein the viral infection is CoViD-19.

4. The AG5 compound for use according to claim 2, wherein the bacterial infection is by bacteria with superantigens.

5. The AG5 compound for use according to claim 2, wherein immunotherapy is carried out by CAR-T cells, by tumor-infiltrating lymphocytes (TIL) or by bispecific antibodies (BiTE).

6. The AG5 compound for use according to any of claims 1 to 5, **characterized in that** it can be administered parenterally or orally.

7. The AG5 compound for use according to any of claims 1 to 6, wherein the compound of formula **I** is administered at a concentration between 0.0001 mg/(kg h) and 10 mg/(kg h) for a time between 1 h and 2000 h.

## Patentansprüche

1. AG5-Verbindung: 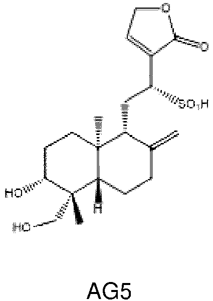 zur Verwendung bei der Behandlung einer Erkrankung, bei der eine mit einem Zytokinsturm verbundene Entzündungsreaktion auftritt.

2. AG5-Verbindung zur Verwendung nach Anspruch 1, wobei der Zytokinsturm durch eine virale Infektion, eine bakterielle Infektion oder eine Immuntherapie verursacht wird.

3. AG5-Verbindung zur Verwendung nach Anspruch 2, wobei die virale Infektion CoViD-19 ist.

4. AG5-Verbindung zur Verwendung nach Anspruch 2, wobei die bakterielle Infektion durch Bakterien mit Superantigenen erfolgt.

5. AG5-Verbindung zur Verwendung nach Anspruch 2, wobei die Immuntherapie durch CAR-T-Zellen, durch tumorinfiltrierende Lymphozyten (TIL) oder durch bispezifische Antikörper (BiTE) durchgeführt wird.

6. AG5-Verbindung zur Verwendung nach einem der Ansprüche 1 bis 5, **dadurch gekennzeichnet, dass** sie parenteral oder oral verabreicht werden kann.

7. AG5-Verbindung zur Verwendung nach einem der Ansprüche 1 bis 6, wobei die Verbindung der Formel I in einer Konzentration zwischen 0,0001 mg/(kg/h) und 10 mg/(kg/h) über einen Zeitraum zwischen 1 h und 2000 h verabreicht wird.

## Revendications

1. Composé AG5 : 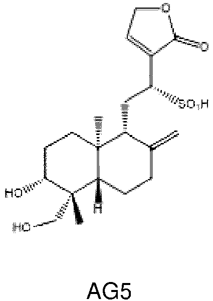 destiné à être utilisé dans le traitement d'une pathologie où une réaction inflammatoire associée à une tempête de cytokines se produit.

2. Composé AG5 destiné à être utilisé selon la revendication 1, dans lequel la tempête de cytokines est provoquée par une infection virale, une infection bactérienne ou une immunothérapie.

3. Composé AG5 destiné à être utilisé selon la revendication 2, dans lequel l'infection virale est le CoViD-19.

4. Composé AG5 destiné à être utilisé selon la revendication 2, dans lequel l'infection bactérienne se fait par des bactéries avec des superantigènes.

5. Composé AG5 destiné à être utilisé selon la revendication 2, dans lequel l'immunothérapie est effectuée par des cellules CAR-T, par des lymphocytes infiltrant la tumeur (TIL) ou par des anticorps bispécifiques (BiTE).

6. Composé AG5 destiné à être utilisé selon l'une quelconque des revendications 1 à 5, **caractérisé en ce qu'**il peut être administré par voie parentale ou orale.

7. Composé AG5 destiné à être utilisé selon l'une quelconque des revendications 1 à 6, dans lequel le composé de formule 1 est administré à une concentration entre 0,0001 mg/(kg h) et 10 mg/(kg h) pendant une durée entre 1 h et 2 000 h.
